(19) Europäisches Patentamt
European Patent Office
Office européen des brevets

(11) EP 0 958 839 B1

(12) **EUROPEAN PATENT SPECIFICATION**

(45) Date of publication and mention
of the grant of the patent:
**29.12.2004 Bulletin 2004/53**

(51) Int Cl.⁷: **A61M 1/34**, A61M 1/36

(21) Application number: **99109637.1**

(22) Date of filing: **06.02.1996**

(54) **Adsorptive extracorporeal removal of cytokines from patients affected with acute organ failure**

Adsorptive extrakoporale Entfernung von Zytokinen bei akutem Organversagen

SEPARATION ADSORPTIVE DE CYTOKINES D'UN PATIENT AFFECTE PAR LA DEFAILLANCE AIGUE D'UN ORGANE

(84) Designated Contracting States:
**DE ES FR GR IT NL**

(43) Date of publication of application:
**24.11.1999 Bulletin 1999/47**

(62) Document number(s) of the earlier application(s) in accordance with Art. 76 EPC:
**96830049.1 / 0 787 500**

(73) Proprietor: **BELLCO S.p.A.**
**20121 Milano (IT)**

(72) Inventors:
• **Wratten Mary Lou**
**41036 Medolla (IT)**

• **Tetta, Ciro**
**41037 Mirandola (IT)**

(74) Representative: **Jorio, Paolo et al**
**STUDIO TORTA S.r.l.,**
**Via Viotti, 9**
**10121 Torino (IT)**

(56) References cited:
**WO-A-90/12632        FR-A- 2 390 965**

• **DATABASE WPI Section Ch, Week 9504 Derwent Publications Ltd., London, GB; Class A96, AN 95-027288 XP002006819 & JP 06 312017 A (ASAHI MEDICAL CO LTD), 8 November 1994 (1994-11-08)**

**Description**

The present invention relates to a highly effective method of purifying blood in patients affected with acute organ failure, and which may be used without difficulty in conjunction with other known blood purifying methods. Though preferably applied to the treatment of acute organ failure, possibly in conjunction with or caused by septic shock, the method according to the present invention may also be used to advantage in improving the effectiveness of known methods of treating patients affected with chronic organ failure.

In patients suffering from organ failure, the blood gradually retains endless quantities of various substances commonly referred to as "toxins", and which, in healthy subjects, the relative organs are normally capable of eliminating from the bloodstream. The accumulation of such toxins, if not treated, results in a wide range of organic malfunctions and eventually in death; and acute syndromes are characterized by a high percentage of cytokines among the toxins for removal.

The most commonly used systems for purifying the blood of toxins are by adsorbing them on solid media (hemo- and plasmaperfusion), or by ultrafiltering the blood or plasma through appropriate semipermeable membranes, either by convection with the aid of a pressure gradient (TMP) through the membrane (hemo- or plasmafiltration), or by diffusion by bringing the blood or plasma to be purified into contact with one side of the membrane, and an appropriately formulated wash solution into contact with the opposite side (hemodialysis).

All the above systems, however, present drawbacks. Hemoperfusion consists in percolating the blood directly through a filter of adsorbent material, which must therefore be made highly biocompatible. This is usually achieved by covering the adsorbent particles with appropriate material, which, however, seriously impairs the toxin-retaining capacity of the particles. In the case of plasmaperfusion, the blood is first filtered to separate the plasma, which is then percolated through the adsorbent material. Though this to some extent solves the problem of biocompatibility during perfusion, the increase in the viscosity of the blood during filtration may result in extensive clotting through the membrane, so that in any case the blood must be treated with anticoagulants (heparin).

Hemo- and plasmafiltration, on the other hand, only provide for removing high-molecular-weight toxins, and produce a considerable weight loss which must be compensated by feeding an infusion solution into the patient's blood. According to EP-A-0451429 the above problem may be partly solved by regenerating the ultrafiltrate, by absorbing the medium-high-molecular-weight toxins in it by percolating it through uncoated-activated-carbon-based hemoperfusion cartridges such as DETOXIL 2™ produced by SORIN BIOMEDICA of Saluggia, Italy, so that the regenerated ultrafiltrate may be used, as it is or with additions, as an infusion solution.

Hemodialysis, particularly if combined with one or more of the above methods, is the most effective, but is relatively lengthy in terms of purification time, and therefore fails to provide for sufficient toxin removal in acute crises. In particular, cytokine removal is fairly poor, so that, at present, organic malfunctions caused by acute organ failure can be no more than delayed as opposed to fully prevented.

It is an object of the present invention to overcome the aforementioned drawbacks by enabling blood to be purified rapidly and effectively of any and all toxins, including cytokines.

According to the present invention, there is provided uncoated activated carbon, a hydrophobic or ion-exchange polystyrene resin, or mixtures thereof as claimed in Claim 1.

A number of non-limiting embodiments of the present invention will be described by way of example with reference to the accompanying drawings, in which:

Figure 1 shows, schematically, a test circuit implementing a device in accordance with the present invention, and used for testing the method according to the invention;

Figures 2 to 7 show test graphs illustrating removal of the principal cytokines in the ultrafiltrate using the Figure 1 circuit.

Roughly speaking, the present invention is based on the observation that uncoated activated carbon - which, according to said EP-A-0451429, has proved a valuable aid in purifying an ultrafiltrate, by adsorption, of toxic substances in general (and $\beta2$microglobulin in particular) of a molecular weight ranging in particular from 300 to 1,500 Dalton (theoretically the adsorption spectrum of activated carbon may range from 100 to 20,000 Dalton) and present in abnormal quantities in patients affected with chronic uremia - may surprisingly also be used - particularly in conjunction with hydrophobic and/or ion-exchange resins - for treating acute patients, to also enable rapid, practically total removal of the class of toxins known as cytokines, and which at present are not effectively removed by any known method or device.

In view of the notoriously adsorbent nature of activated carbon within the above range of molecular weights, it may be considered obvious that at least part of the cytokines would be retained by an activated-carbon filter, the molecular weight of the principal known cytokines ranging between 9,000 and 17,000 Dalton (only IL-6 has a molecular weight of 26,000 Dalton, i.e. well outside the known adsorption range of activated carbon). To achieve the objects of the present invention, however, as opposed to merely capturing at least part of the cytokines present in the bloodstream of acute patients, it is of vital importance to eliminate them as rapidly as possible.

Up to now, no indication has been given in existing literature that activated carbon - especially if used, as will be seen, in conjunction with resins - is also capable of ensuring sufficiently rapid removal of cytokines to permit effective treatment of acute patients. Thanks to the insight of the Applicant's researchers, however, this capacity has now been brought to light, and has been optimized by treating the bloodstream of acute patients according to a specific sequence. To begin with, ultrafiltration or plasmafiltration is performed, whereby the stream of blood to be purified is brought into contact with a first side of a known semipermeable membrane, e.g. a polysulfone membrane (PSF, SPAN), through which a pressure gradient (TMP) is maintained to collect by convection, on a second side of the membrane opposite the first, a stream of ultrafiltrate or plasmafiltrate, which is shunted to a secondary circuit, while the rest of the stream of blood (eluate) is removed.

The above step is performed using a membrane of such physical-chemical and permeability characteristics (porosity, hydrophobicity, etc.) as to permit most (substantially all) of the cytokines present in the incoming blood stream to pass through the membrane into the ultrafiltrate/plasmafiltrate stream. The ultrafiltrate/plasmafiltrate stream containing the cytokines to be eliminated is then fed by said secondary circuit through filtering means forming part of the secondary circuit and so located that the stream containing the cytokines to be eliminated is brought into contact with uncoated activated-carbon granules, e.g. inside a first filter cartridge (adsorption column), as well as with granules of a hydrophobic or ion-exchange resin mixed with the carbon granules or, preferably, inside a second filter cartridge (adsorption column) hydraulically in series with the first.

This provides not only for rapidly eliminating the cytokines, which are retained in the two cartridges, thus achieving the object of the present invention, but also, as described in EP-A-0451429, for regenerating the ultrafiltrate/ plasmafiltrate (activated carbon is also capable of retaining any other medium-molecular-weight toxins such as hippuric acid, uric acid, β2microglobulin, etc.) which may thus be used as a reinfusion solution and added to the eluate from the ultrafilter/plasmafilter to form a stream of purified blood which is then fed back in known manner into the patient's body.

According to the invention, the cytokines are removed more rapidly and effectively (and, above all, the life of the filter cartridges of the secondary circuit is extended) if the activated carbon is also used in conjunction with a hydrophobic resin, e.g. resins with a reticulate aromatic polymer base, macroreticulate and/or macroporous resins, such as those known commercially as AMBERLITE™ (styrene-methacrylate resin) and AMBERCHROME™ (copolymer divinylbenzene-polystyrene resin), and/or a synthetic carbonaceous resin such as AMBERSORB™. All these resins, which are produced and marketed by ROHM&HAAS and normally used in the manufacture of fruit juices and other food products, conform with and are classified nontoxic by American FDA Standards nr 21 CFR 173.65, and may therefore also be employed for medical purposes. Nevertheless, it still remained to be discovered that such resins (which are normally used as fillers for chromatography columns or as immobilizing supports for active substances) were also effective - and moreover, as they are - in retaining cytokines, and, what is more, at such a rate as to enable them to be used in the treatment of acute organ failure syndromes.

The resins used in the present invention have a granule size of 20 to 100 (250-350 for AMBERLITE™) micron (thousandths of a millimeter), and a porosity of 100 to 300 Angstrom for AMBERCHROM™, 30 to 480 Angstrom for AMBERSORB™, and 130 to 150 Angstrom for AMBERLITE™.

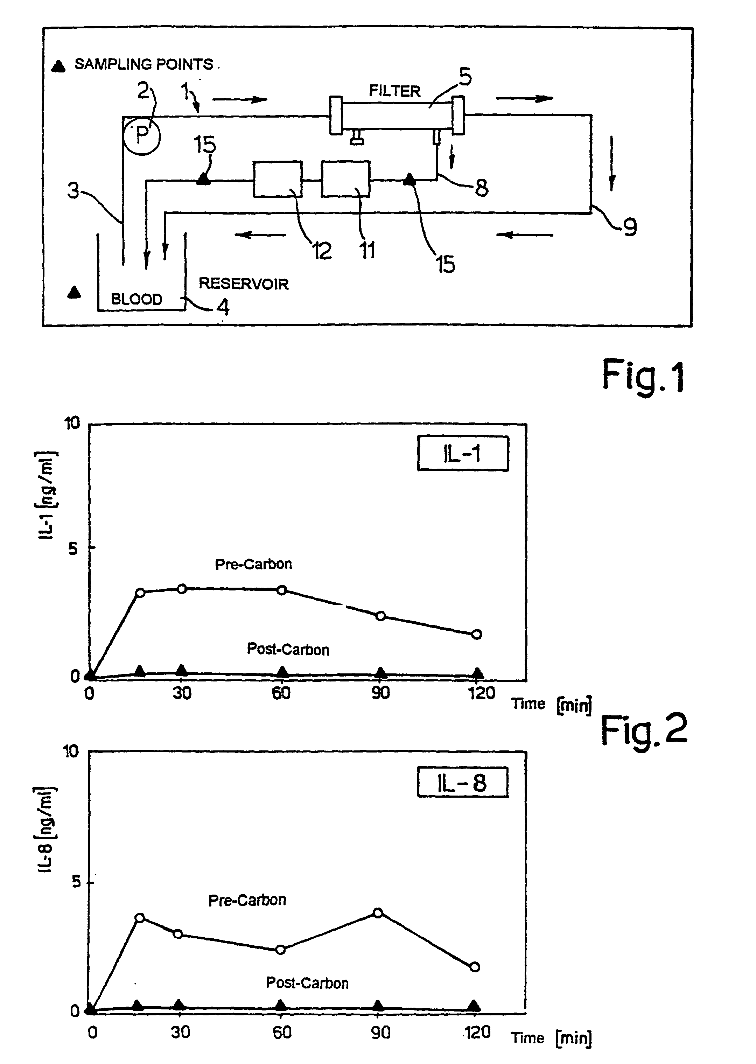

With reference to Figure 1, the method above has been tested in the circuit shown, and may be implemented in the device described in EP-A-0451429 by simply replacing the single uncoated-activated-carbon cartridge (DETOXIL-2™ by SORIN Biomedica of Saluggia) with two separate hemoperfusion cartridges hydraulically in series with each other and respectively packed with activated carbon and with one (or a mixture) of the above resins. The main components of such a device are already shown in the Figure 1 circuit, which comprises a main hydraulic circuit 1 presenting a circulating pump 2 and means for supplying a stream of blood to be purified, and which, in the example shown, are defined by a branch tube 3 immersed inside a reservoir 4 filled with a predetermined quantity of blood for treatment. Circuit 1 is connected hydraulically in series with a filter (ultrafilter or plasmafilter) 5 presenting a filtering means defined by a semipermeable membrane, and which provides for filtering the stream of blood by convection to form a stream of ultrafiltrate/plasmafiltrate inside a secondary circuit 8 branching hydraulically from circuit 1. The ultrafiltrate/plasmafiltrate flows along circuit 8 and is combined with a stream of eluate, issuing from filter 5 along a discharge tube 9, to form a stream of purified blood, which may be fed back using known means into the patient's body. For test purposes, in the example shown, circuit 8 and tube 9 both discharge into reservoir 4, so that the blood in the reservoir is batch treated cyclically for a predetermined length of time. Secondary circuit 8 presents two filters hydraulically in series with each other, a first defined by a hemoperfusion cartridge 11 packed with uncoated activated carbon, and a second defined by a similar cartridge 12 packed with one (or more) of the aforementioned resins. Sampling points 15 are provided up- and downstream from filters 11, 12 to monitor purification of the blood in reservoir 4.

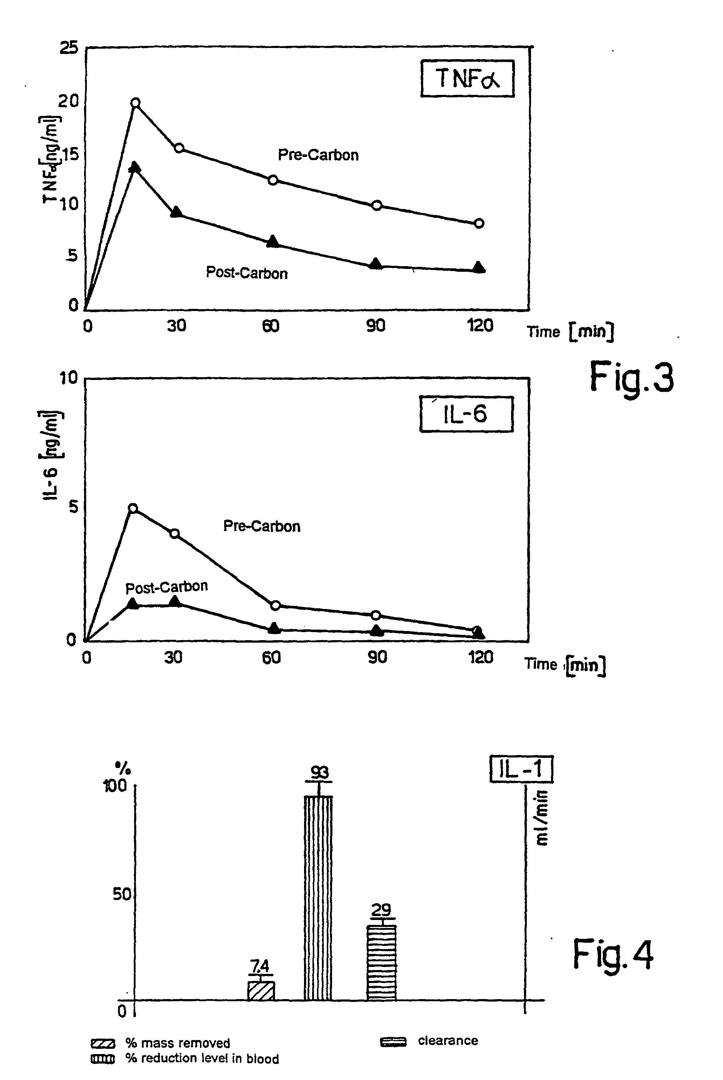

As shown in the Figure 2 to 7 graphs, and as described in more detail later on, the present invention provides for a rapid, steady reduction in the cytokines present in the blood in reservoir 4. A number of practical embodiments of the present invention will now be described by way of example.

EXAMPLE 1

- Characterization of adsorption on activated carbon and resins:

Uncoated activated carbon and the resins listed in Table 1 are packed, prewashed, by gravity into small columns (2.5 cm long by 1.2 cm inside diameter) formed from polystyrene tubes, to a total weight of 1.3 g. The resins are extensively washed and stored in a 50% solution of methanol and water. Immediately before perfusion, the cartridges so formed are washed with 20 times the volume of the cartridge (20 volumes) of sterile physiological saline solution and then conditioned with human plasma (10 ml). Normal human plasma is then prepared containing 2-2.5 $\mu$Ci (microcurie) of $^{125}$I labelled cytokines, such as TNF-$\alpha$ (NEN-Du Pont, specific activity 59.2 $\mu$Gi/mg), IL-1$\beta$ (Amersham, specific activity 3000 Ci/mmol), IL-6 (Amersham, specific activity 1100 Ci/mmol) and IL-8 (Amersham, specific activity 2000 Ci/mmol). Unlabelled cytokines are added to 100 ml of stirred plasma to the indicated concentration, and specifically 500 pg/ml for TNF-$\alpha$, 20 ng/ml for IL-1$\beta$, 50 ng/ml for IL-6, and 20 ng/ml for IL-8. After extensive stirring, 0.2 ml of plasma containing cytokines are counted in a $\gamma$-scintillation counter. Each cytokine is studied separately in a sterile single-pass circuit of the type in Figure 1, in which branch 8 is absent and filter 5 comprises the (resin or carbon) cartridge prepared as described above. The primary volume of the circuit used is 5 ml, and the blood flow rate through the test cartridge is maintained at 30 ml/hour. Aliquots of 0.2 ml are taken downstream from the test cartridge every 30 minutes. The amount of free iodine in the plasma samples is determined using the perchloric acid method and radioactivity counting in the supernatant. Perfusion is arrested when the radioactivity c.p.m. in the samples taken downstream from the cartridge equals that in the basal samples, thus indicating saturation of the cartridge. The cartridges are then disconnected from the circuit, washed in 20 volumes of isotonic saline solution, and opened. Aliquots of each tested adsorbent product are dried on filter paper, weighted and placed in tubes for counting. The results are expressed in ng of cytokine bound to 1 g of each adsorbent product, according to the following formula:

(1)     [(cpm bound/cpm basal)/mg of weighted sample]x100

The results are shown in Table 1.

TABLE 1

| Adsorbent | TNF (ng/g) | IL-1 (ng/g) | IL-8 (ng/g) | IL-6 (ng/g) |
|---|---|---|---|---|
| Amberlite™ | 176 | 60 | 69 | 324 |
| Amberchrom™ | 395 | 235 | 725 | 168 |
| Ambersorb™ | 310 | 636 | 574 | 171 |
| Activ carbon | 71 | 320 | 100 | 168 |

EXAMPLE 2

- Filtration/adsorption in an in vitro recirculating model

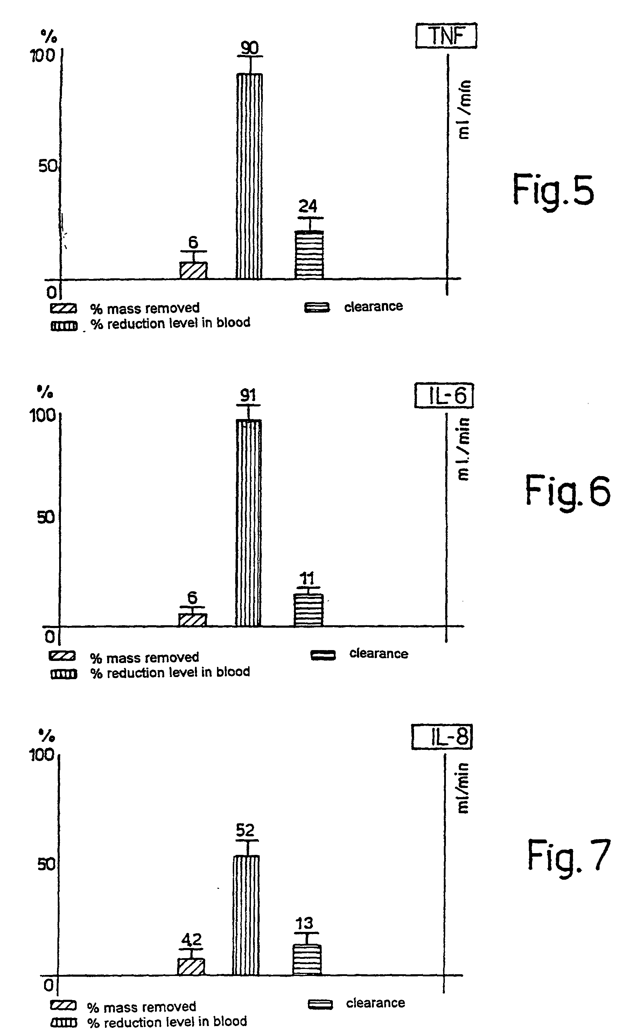

Four hundred (400) ml of fresh human blood drawn in ACD are added to 5 mg of LPS and incubated for 4 hours at 37°C. The blood is then recalcified, added to 5 U/ml of heparin (Liquemin, La Roche, Basle, Switzerland), and circulated in a closed-loop model as in Figure 1. An ultrafilter (BELLCO S.p.A. HFTO4 0.5 m$^2$ polysulfone membrane) is connected to the blood-side compartment of reservoir 4 containing the LPS-challenged blood. The ultrafiltrate is fed along branch 8 fitted with a single adsorbent cartridge 11 or 12 of 130 g of activated carbon or resin. After passing through the adsorbent cartridge, the ultrafiltrate is recirculated into reservoir 4. The blood flow rate in the main circuit is maintained at 200 ml/minute and the ultrafiltrate flow rate at 30 ml/minute by controlling the transmembrane pressure (TMP), and the duration of recirculation for each test is 120 minutes. Samples to determine IL-1$\beta$, TNF-$\alpha$, IL-8 and the receptor antagonist of IL-1 (IL-1ra) are taken upstream from the ultrafilter (as "blood levels") at different time intervals, i.e. after 0, 5, 15, 30, 60, 90 and 120 minutes; and further samples are taken up- and downstream from the adsorbent cartridge (points 15) at time intervals of 0, 15, 30, 60, 90 and 120 minutes. The blood samples are immediately centrifuged at 1000 rpm for 20 minutes at 4°C, and the recovered plasma is stored at -80°C until essayed to determine IL-1β, TNF-α, IL-8 and the receptor antagonist of IL-1 (IL-1ra) using commercial ELISA kits. The results are shown in Figures 2 to 7. As can be seen, the resins not only provide for greater overall cytokine adsorption as compared with activated carbon (Table 1), but also for more rapidly eliminating high cytokine levels from the blood, as shown both by the high removal percentages and by the good clearance levels. As shown in Figures 2 and 3, however, activated carbon alone also provides for excellent cytokine removal from the ultrafiltrate, which is limited only towards TNF-α, which, however, is largely retained (Figure 5) by the resins (90% elimination level and clearance of 24). Ideal application of the present invention therefore consists in double adsorption of the ultrafiltrate, first on carbon and then on resin, possibly selecting a specific resin (or combination of different resins) depending on the levels of the various cytokines to be absorbed, to ensure adequate working life of cartridges 11 and 12.

## Claims

1. Use of uncoated activated carbon, a hydrophobic or ion-exchange polystyrene resin, or mixtures thereof for the preparation of a medicament for the treatment of a patient affected with acute organ failure by adsorptive removal of at least one cytokine selected from the group consisting of: IL-1, IL-6, IL-8, TNF and mixtures thereof contained in an ultrafiltrate or plasmafiltrate derived from blood of said patient.

2. Use as claimed in Claim 1, **characterized in that** said hydrophobic resins are selected from the styrene-methacrylate and copolymer divinylbenzene-polystyrene group of resins.

3. Use as claimed in Claim 1 or 2, **characterized in that** said ion-exchange resins are selected from the synthetic carbonaceous group of resins.

4. Use as claimed in one of the foregoing Claims from 1 to 3, **characterized in that** the hydrophobic synthetic resins are of the AMBERLITE™, AMBERCHROM™ or AMBERSORB™ type.

5. Use as claimed in one of the foregoing Claims from 1 to 4, **characterized in that** said resins present a granule size of 20 to 350 micron, preferably of 10 to 100 or 250 to 350 micron, and a porosity of 100 to 300 Angstrom.

## Patentansprüche

1. Verwendung eines nicht umhüllten, aktivierten Kohlenstoffs, eines hydrophoben oder Ionenaustausch-Polystyrolharzes, oder Mischungen derer zur Herstellung eines Medikaments zur Behandlung eines Patienten, welcher von akutem Organversagen betroffen ist, durch das adsorptive Entfernen mindestens eines Cytokins, welches aus der Gruppe, die aus IL-1, IL-6, IL-8, TNF und Mischungen derer besteht, ausgewählt ist, das in einem Ultrafiltrat oder einem Plasmafiltrat, welches aus dem Blut dieses Patient stammt, erhalten wird.

2. Verwendung gemäß Anspruch 1, **dadurch gekennzeichnet, daß** diese hydrophoben Harze aus der Styrol-Methacrylat- und Copolymer Divinylbenzol-Polystrol-Gruppe der Harze ausgewählt sind.

3. Verwendung gemäß Anspruch 1 oder 2, **dadurch gekennzeichnet, daß** diese Ionenaustausch-Harze aus der synthetischen, kohlenstoffhaltigen Gruppe der Harze ausgewählt sind.

4. Verwendung gemäß einem der vorhergehenden Ansprüche 1 bis 3, **dadurch gekennzeichnet, daß** die hydrophoben synthetischen Harze vom Typ AMBERLITE™, AMBERCHROM™ oder AMBERSORB™ sind.

5. Verwendung gemäß einem der vorhergehenden Ansprüche 1 bis 4, **dadurch gekennzeichnet, daß** die Harze eine Granulatgröße von 20 bis 350 μm, vorzugsweise von 10 bis 100 μm oder 250 bis 350 μm, aufweisen, und eine Porosität von 100 bis 300 Å (Angstrom).

## Revendications

1. Utilisation d'un charbon actif non enduit, d'une résine de polystyrène hydrophobe ou échangeuse d'ions, ou de mélanges de ceux-ci pour la préparation d'un médicament pour le traitement d'un patient atteint d'une insuffisance aiguë d'un organe à l'aide du retrait adsorbant d'au moins une cytokine choisie dans le groupe constitué par : l'IL-1, l'IL-6, l'IL-8, le TNF et les mélanges de ceux-ci contenus dans un ultrafiltrat ou un plasmafiltrat dérivé du sang dudit patient.

2. Utilisation selon la revendication 1, **caractérisée en ce que** lesdites résines hydrophobes sont choisies parmi le groupe de résines de styrène-méthacrylate et du copolymère de divinylbenzène et de polystyrène.

3. Utilisation selon la revendication 1 ou 2, **caractérisée en ce que** lesdites résines échangeuses d'ions sont choisies parmi le groupe de résines synthétiques carbonées.

4. Utilisation selon l'une des revendications précédentes 1 à 3, **caractérisée en ce que** les résines synthétiques hydrophobes sont de type AMBERLITEMD, AMBERCHROMMD ou AMBERSORBMD.

5. Utilisation selon l'une des revendications précédentes 1 à 4, **caractérisée en ce que** lesdites résines présentent une granulométrie de 20 à 350 $\mu$m, de préférence de 10 à 100 ou de 250 à 350 $\mu$m, et une porosité de 100 à 300 Å.

**Fig.1**

**Fig.2**

Fig.3

Fig.4

Fig. 5

Fig. 6

Fig. 7